# EUROPEAN PATENT SPECIFICATION

(11) **EP 3 257 435 B1**
(45) Date of publication and mention of the grant of the patent: **09.06.2021**
(21) Application number: 17020241.0
(22) Date of filing: 02.06.2017
(51) Int. Cl.: A61B 3/113, A61B 5/00, A61B 3/14

(54) **ARRANGEMENT FOR EYE MOVEMENT DETECTION AND CALIBRATION METHOD**
ANORDNUNG ZUR AUGENBEWEGUNGSDETEKTION UND KALIBRIERVERFAHREN
AGENCEMENT POUR LA DÉTECTION DE MOUVEMENT OCULAIRE ET PROCÉDÉ D'ÉTALONNAGE

(30) Priority: 08.06.2016 SE 1630144
(43) Date of publication of application: 20.12.2017
(73) Proprietor: BalansDiagnos AB, 11264 Stockholm (SE)
(72) Inventor: GEISLER, Christian, 11264 Stockholm (SE)
(74) Representative: Heimdal, Pär

(56) References cited:
- WO-A1-2016/001902
- JP-A- 2013 031 631
- US-A1- 2007 121 068
- US-A1- 2015 223 683

## Description

### TECHNICAL FIELD

This document discloses an arrangement and a method. More particularly, an arrangement for detecting eye movement of an eye is described, and a calibration method of the described arrangement.

### BACKGROUND

Vertigo and dizziness are very common symptoms in general healthcare. There are several cause for this symptom.

According to several scientific investigations, a vestibular cause is expected in 50% of all cases of vertigo and dizziness; 20-25% have a psychogenic cause, and in 25% of all cases "something else" is causing the symptoms (Kroenke 2000). "Something else" may refer to e.g. blood pressure- cardiac- or drug-related causes, or intoxications. In a specialised dizzy clinic, it is also usual to find patients with more than one single cause explaining their symptoms. Especially a compound of vestibular and psychogenic causes is a common mix.

The nerve impulses of the vestibular system are connected with the eye muscles. To look for eye movements is a window into the vestibular system. Provocation of a series of standardised vestibular impulses will lead to a standardised pattern of eye movements, also called nystagmus. A skilled investigator is able to judge whether the provoked eye movements are expected and physiologic or if the movements are abnormal. For diseases involving the vestibular system it is crucial to be able to judge whether the provoked eye movements are normal or not.

The five most usual dizzy diseases are believed to explain 75% of all vertigo and dizziness (Brandt 1999). These are Benign Paroxysmal Positional Vertigo (BPPV), vestibular neuritis, vestibular migraine, Ménière's disease and Phobic postural vertigo. Four of these diseases involve the vestibular system and will lead to pathological eye movement during a standardised clinical evaluation if the patient is investigated when having symptoms.

Though diagnostic nystagmus may be expected in at least 50% of all patients complaining about vertigo, dizziness and balance symptoms, in general health care the vestibular system is often not investigated at all. This is due to lack of technical equipment to capture eye movements and because of lack of knowledge to judge them.

When having a vertigo attack the patient is seldom in reach of medical facility. At the moment when the patient reaches the skilled investigator, there may be no more diagnostic eye movements for the physician to analyse. The patient, when actually having a vertigo attack may also be so dizzy that visiting the hospital, or even leaving home may be difficult.

It would for this reason be beneficial for the patient, in order to get his/ her vertigo diagnosed correctly, to find a way of capturing eye movements of the patient at the moment when the vertigo occurs, preferably at the location of the patient, and somehow allow the physician to perform a diagnosis of the eye movements.

In that context, documents JP2013-31631 A, US2007/121068 A1, US2015/223683 A1 and WO2016/001902 A1 disclose prior art arrangements comprising a headset and a camera to take a sequence of pictures of at least an eye of a patient.

### SUMMARY

It is therefore an object of this invention to solve at least some of the above problems and enable diagnosis of eye movements of a patient by detecting and capturing eye movements of the patient.

According to a first aspect of the invention, this object is achieved by an arrangement according to claim 1 for examining eye movements of a first eye of a patient via a camera.

According to a second aspect of the invention, this object is achieved by a method according to claim 5 for calibrating an arrangement according to the first aspect, for enabling examination of eye movements of a first eye of a patient via a camera.

Thanks to the described aspects video sequences may be captured of eye movements, suspected to be associated with a vertigo attack of a patient, at the moment of.

By calibrating the arrangement according the provided second aspect, it is possible for the patient to ascertain that the images are captured with a satisfying quality for enable the physician to diagnose the patient, based on the images. Thereby, diagnosis of eye movements of a patient is enabled.

Other advantages and additional novel features will become apparent from the subsequent detailed description.

### FIGURES

Embodiments of the invention will now be described in further detail with reference to the accompanying figures, in which:
- **Figure 1A**: illustrates an arrangement according to an embodiment of the invention, as seen from the front;
- **Figure 1B**: illustrates the arrangement according to an embodiment of the invention, as seen in profile;
- **Figure 2**: illustrates the arrangement, according to an embodiment, as seen from above;
- **Figure 3A**: illustrates the arrangement according to an embodiment, as seen from the inside;
- **Figure 3B**: illustrates a headset of the arrangement according to an embodiment;
- **Figure 4**: is a flow chart illustrating an embodiment of a method.

### DETAILED DESCRIPTION

Embodiments of the invention described herein are defined as an arrangement and a method, which may be put into practice in the embodiments described below. These embodiments may, however, be exemplified and realised in many different forms and are not to be limited to the examples set forth herein; rather, these illustrative examples of embodiments are provided so that this disclosure will be thorough and complete.

Still other objects and features may become apparent from the following detailed description, considered in conjunction with the accompanying drawings. It is to be understood, however, that the drawings are designed solely for purposes of illustration and not as a definition of the limits of the herein disclosed embodiments, for which reference is to be made to the appended claims. Further, the drawings are not necessarily drawn to scale and, unless otherwise indicated, they are merely intended to conceptually illustrate the structures and procedures described herein.

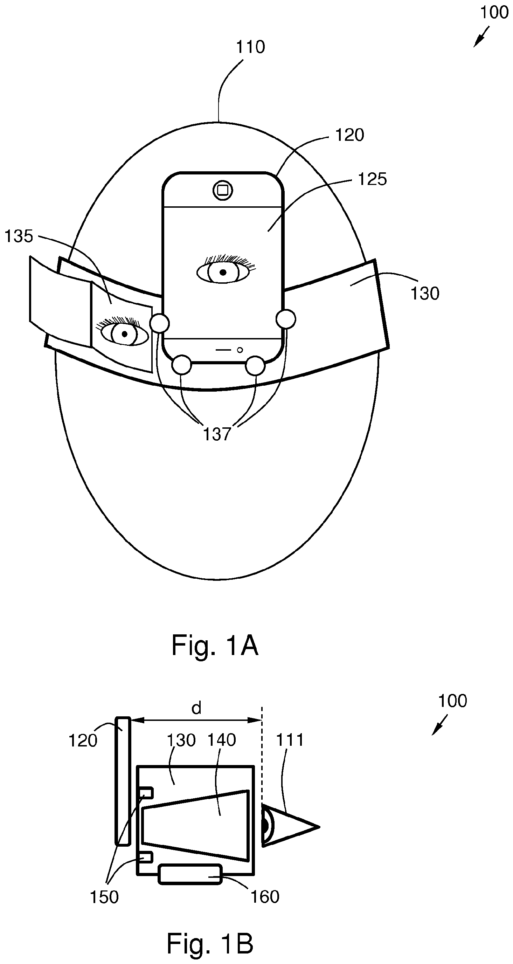

**Figure 1A** illustrates a scenario with an arrangement **100** situated on a head **110** of a patient.

The purpose of the arrangement 100 is to enable examination of eye movements of a first eye **111** of the patient via a camera **120,** comprised in the arrangement 100. The camera 120 is held in an appropriate position for taking a sequence of pictures of the first eye 111 by a headset **130.**

The headset 130 is configured to fit on the head 110 of the patient, e.g. by a fastening means such as a set of strings, straps, belts or similar, which may be elastic and/ or comprise a Velcro fastening arrangement, or other corresponding fastening means.

Further, the headset 130 is configured to hold and fixate the camera 120 in a position where a lens of the camera 120 is directed towards the first eye 111 of the patient, at a distance d from the first eye 111. The camera 120 is comprised in a mobile telephone, such as e.g. the mobile telephone of the patient, in some embodiments.

The headset 130 further comprises a closable opening 135 in front of a second eye of the patient, which second eye is not examined by the camera 120.

The reason why only pictures are captured of one eye of the patient, i.e. the first eye 111, is that the eye movements of the patient (or any other person) are synchronised. For this reason, it is enough to examine only one eye, in order to make a diagnosis. Thereby, only one camera 120 is required, which keeps down the costs of the arrangement 100, the complexity of the headset 130, the weight of the arrangement 100 and the amount of data/ images to capture, store, transfer to the physician and analyse, by the physician, is reduced with 50% in comparison with analysing both eyes of the patient.

The arrangement 100 is used by the patient to document the *nystagmus* (rapid involuntary movements of the eyes) during a vertigo attack. When using the arrangement 100, according to some embodiments, a sequence of test instructions may be provided to the patient, e.g. in form of a recorded voice message replayed and emitted via a loudspeaker. The sequence of test instructions may be patient specific and may comprise a certain pattern of movement of the head or eyes, while the camera 120 is used for video recording.

The resulting documentation, i.e. a short movie sequence or sequence of images may then be communicated to the physician who in turn can make a diagnosis whether the patient vertigo is due to defects in the balance organ or not, based on knowledge about expected eye movements during those certain tests.

In order to use the arrangement 100 and be able to capture images of the eye movements, the camera lens must be correct positioned relative the patient eye 111.

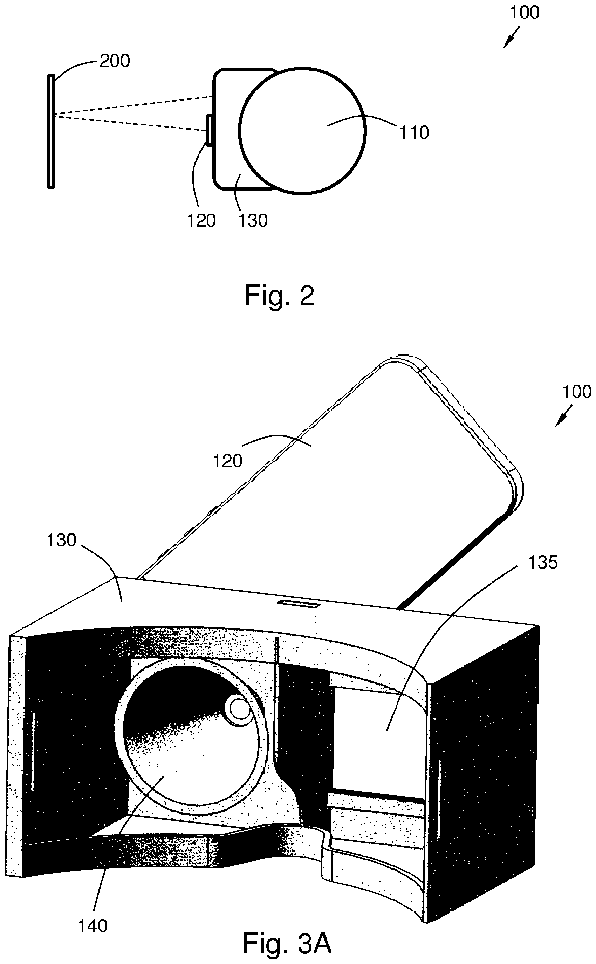

The eye 111 must also be sufficiently illuminated during video recording. This may be made in various ways; using artificial visible or infrared light, or natural light, as will be further discussed together with the presentation of Figure 2B.

Finally, in most embodiments, it is important that the patient is not able to focus any of his/ her eyes during the examination, i.e. when performing the sequence of test instructions with the arrangement 100. Otherwise, the eye movements of the patient may not behave as expected according to the diagnosis method. It may be avoided that the eyes are focused e.g. by keeping the headset interior dark, by applying a diffusion lens before any or both of the patient's eyes, etc.

As already stated, if the patient is able to focus one of the eyes, it will influence the eye movements also of the other eye as eye movements are correlated. At the same time, it is important that the patient is able to somehow see and position the camera 120 in the headset 130 correctly, and also position the headset 130 correctly on the head 110, and be certain that the camera 120 is directed to and capture images of the eye 111.

In the disclosed headset 130, a closable opening 135 is arranged, at the position of the patients second eye, which will not be examined.

Thereby the patient is enabled to, when putting on the headset 130 and opening the closable opening 135, confirm that the camera 120 is in position and via a mirror he/ she may observe an image of the first eye 111, in a display 125 of the camera 120, with the second eye through the opening 135 in the headset 130. If necessary, he/ she may also adjust the arrangement 100 so that the image captured of the first eye 111 is centred and focused on the first eye 111, before the examination program exercises starts. When the examination starts, he/ she may close the opening 135 and follow the provided instructions.

The headset 130 may comprise a mounting device **137** to fixate the camera 120. The mounting device 137 may comprise a fastener, screw joints, Velcro, etc., in different embodiments.

The camera 120, may e.g. be a digital camera with video capacity. The camera 120 may be built in in a mobile telephone in some embodiments. An advantage therewith is that most people/ patient has, or at least has access to a mobile telephone with an in-built camera, which may be used for the herein described purpose. Thereby also the price for the arrangement 100 is brought down considerably, as the patient only has to be provided with the headset 130. The aforementioned instructions for the patient may be downloaded as an app.

Another advantage is that the captured images of the eye 111 may be sent to the physician via the mobile telephone. Thereby time is saved and the patient could be diagnosed relatively fast, possibly even in real time.

However, in other embodiments, any camera 120 capable of capturing a sequence of images may be utilised, such as a digital video camera. Further, captured images may be stored in a memory of the camera 120, or the mobile telephone as the case may be, which may be sent or brought to the physician.

The optional memory may comprise a tangible, physical device utilised to store data or programs, i.e., sequences of instructions, on a temporary or permanent basis. According to some embodiments, the memory may comprise integrated circuits comprising silicon-based transistors. The memory may comprise e.g. a memory card, a flash memory, a USB memory, a hard disc, or another similar volatile or non-volatile storage unit for storing data in a nontransitory manner such as e.g. ROM (Read-Only Memory), PROM (Programmable Read-Only Memory), EPROM (Erasable PROM), EEPROM (Electrically Erasable PROM), etc., in different embodiments.

In yet other embodiments, the captured sequence of images of the eye 111 may be sent to the physician as a communication attachment, e.g. e-mail attachment, or streamed to the physician which in turn may store the captured sequence of images of the eye 111 in a database, associated with an identity reference of the patient.

**Figure 1B** is a side view cross section illustrating an exemplary embodiment of the arrangement 100. The headset 130 holds and fixates the camera 120 at a distance **d** from the first eye 111 of the patient. The distance d is at least longer than the minimum focus distance for the camera 120, such as e.g. about 2 - 7 cm, etc., or somewhere in between. This distance may be predetermined and may be different for different cameras 120/ mobile telephone models.

Further, in some embodiments, the arrangement 100 also comprises a light source **150,** situated in the headset 130, configured to illuminate the first eye 111 of the patient. The light source 150 may comprise e.g. a set of Light-Emitting Diodes (LEDs) in some embodiments. Such set of LEDs may comprise one or more diodes emitting light in the same or different colours. Thereby, emission of white light may be achieved, e.g. by using one blue LED diode, one green LED diode and one red LED diode. Another possibility of emitting white light may be to combine blue LED and yellow phosphor. Yet another option may be to use homoepitaxially grown zinc selenide (ZnSe) on a ZnSe substrate that simultaneously emitted blue light from its active region and yellow light from the substrate.

The light source 150 may in other embodiments comprise Organic Light-Emitting Diodes (OLEDs), Quantum dot LEDs (QD), multicolour LEDs, incandescent light bulbs, Xenon flash lamps, plasma lamps, neon/ argon lamps, arc lamps, compact fluorescent lamps, etc., or a combination of at least some of these exemplary light sources.

The light source 150 may in some embodiments also comprise natural light, allowed to enter through an opening in the headset 130, and/ or an arrangement external light source, allowed to enter through said opening, in some alternative embodiments.

The light source 150 may also, or alternatively, comprise fluorescent emitting elements, e.g. by applying strongly fluorescent pigments on the inside of the headset 130 in some embodiments.

In some alternative embodiments, a light source of the camera 120, or the mobile telephone, may be utilised for illuminating the eye 111.

Further, the arrangement may comprise an energy source 160, for feeding the light source 150 with current. The energy source 160 may comprise e.g. a battery, a fuel cell, a photovoltaic cell, etc. However, the light source 150 may in some embodiments be fed with current from an energy source external to the arrangement 100.

The light source 150 may be activated/ deactivated by the user via a switch. Alternatively, a switch may be situated in the headset 130, at the interface between the headset 130 and the head 110 of the patient.

The arrangement 100, and more in particular, the headset 130 may comprise a truncated cone **140,** to be situated in front of the first eye 111 of the patient; i.e. the eye 111 to be examined and documented/ photographed by the camera 120. The wide opening of the truncated cone 140 may be directed towards the first eye 111 and the narrow opening of the truncated cone 140 directed towards the lens of the camera 120.

The functionality of the truncated cone 140 is to enable illumination of the eye 111, by the light source 150, without the eye 111 being given any light to focus on; or to be dazzled by, for that matter. In case the eye 111 is allowed to focus on a particular point, the eye movement that is desired to be captured on the stream of images may be withheld.

Further, the headset 130 may be configured to be attached to the patient's head 110, screening off external light from falling into the first eye 111 of the patient. Thereby, by shielding off external light from falling into the first eye 111 of the patient, it is avoided that the eye 111 is enabled to focus on any particular object, which stimulates the eye movements that is desired to record and analyse.

In some embodiments, a dioptre lens, such as e.g. a strong dioptre lens of 20 or more dioptres may be added between the camera and the eye 111 of the patient. It may thereby be assured that the eye 111 is disabled from focusing on any particular object in the headset 130, as such focusing may disturb the examination of the patient.

**Figure 2** illustrates the scenario of Figure 1A, as it may be seen from above when the patient is calibrating the arrangement 100.

The headset 130 may, in some embodiments, comprise a mounting device 137 to fixate the camera 120 so that a display 125 of the camera 120 is visible for the patient when looking in a mirror **200** with the second eye through the closable opening 135 in the headset 130.

The calibration of the arrangement 100 may ascertain that the image captured of the first eye 111 by the camera 120, is centred and focused on the first eye 111. It is thereby ascertained that the physician will be able to analyse and examine the eye movements correctly.

Thanks to the closable opening 135 in the headset 130 and the mirror 200, the patient is enabled to calibrate the arrangement 100 for enabling examination of eye movements of a first eye 111 of a patient via a camera 120.

Thereby, the patient may activate the camera 120 and observe an image of the first eye 111, in a display 125 of the camera 120, with the second eye via the mirror 200, through the opening 135 in the headset 130. The patient may then adjust the arrangement 100 so that the image captured of the first eye 111 is centred and focused on the first eye 111.

**Figure 3A** illustrates the arrangement 100 according to an embodiment, as regarded from the inside of the arrangement 100.

The truncated cone 140 of the headset 130 is here illustrated from the inside, illustrating how it seals of the eye 111 from visual impact.

In the illustrated embodiment, the camera 120 is integrated in a mobile telephone, mounted on the frontal part of the headset 130 in a tilted manner in order to keep balance of the headset 130, avoiding it to slide over to one side of the head 110.

The headset 130 may be made of cardboard in some embodiments, which is folded by the user, according to instructions. Thereby, the headset 130 may be sent to the patient in a relatively flat mail by a post service, to which the patient then attaches his/ her mobile telephone to the headset 130, using it as camera 120. The cardboard cone 140 may be glued to the headset 130 during the manufacturing. The mobile telephone/ camera 120 may be fixated to the headset 130 by a sticky surface in some embodiments. The headset 130 may comprise slots for head straps on the sides and the upper side of the headset 130.

In other embodiments, the headset 130 may be made of cardboard, but with a front plane made in plastic. The plastic front plane may comprise the cone 140, the sticky surface and the closable opening 135 for the other eye which may be covered with e.g. a rectangular piece of plastic, cardboard or similar. The plastic front part may be intended for reuse while the rest of the headset 130, made of cardboard, may be disposed along with the face padding and head strap.

In yet some embodiments, the sufficient distance d between the camera 120 and the eye 111 may be created by letting the headset 130 be part of the same plastic mold. The casing is intended for reuse by having the patient send the headset 130 back to the clinic, where it is wiped off with e.g. alcohol or similar means, and then be reused by another patient, possibly with a new head strap and face padding.

In yet some alternative embodiments, the headset 130 may be formed in 3D printed plastic; carbon fibre, glass fibre, metal, etc.

The head straps which are attached to the headset 130, e.g. in slots, may be elastic in order to fit better on the patient's head 110 and keep the arrangement 100 in place also when the patient is moving the head 110, or holding the head 110 in bended positions.

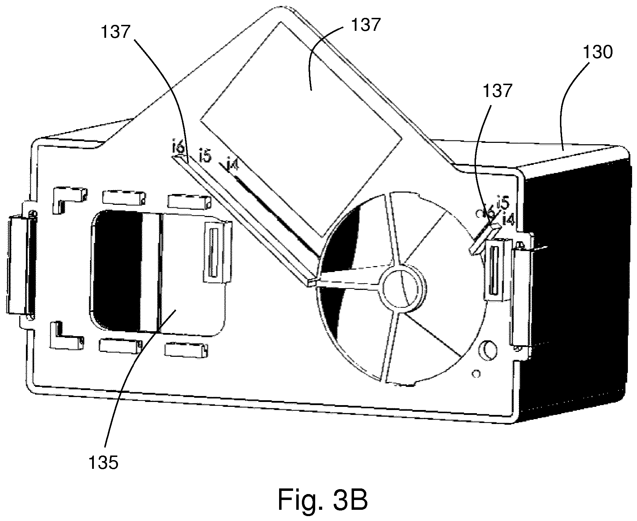

**Figure 3B** illustrates the headset 130 according to an embodiment, as regarded from the front.

The headset 130 comprises a mounting device 137 to fixate the camera 120, or the mobile telephone comprising the camera 120, so that a display 125 of the camera 120/ mobile telephone is visible for the patient when looking in a mirror 200 with the second eye through the closable opening 135 in the headset 130.

The mounting device 137 may comprise one or a plurality of shelves for holding the camera 120, e.g. in combination with a strap, e.g. elastic strap with Velcro. Also, or alternatively, the camera 120 may be hold by screw joints holding a perforated plate, thereby creating a pocket for holding and fixating the camera 120 to the desired position so that capture of images of the patients eye 111 is enabled through the opening of the truncated cone 140, in some embodiments.

The mounting device 137 may in some embodiments comprise other means for fixating the camera 120 at the headset 130, e.g. based on magnets, adhesive tape, pins, spring means, resilient members, etc.

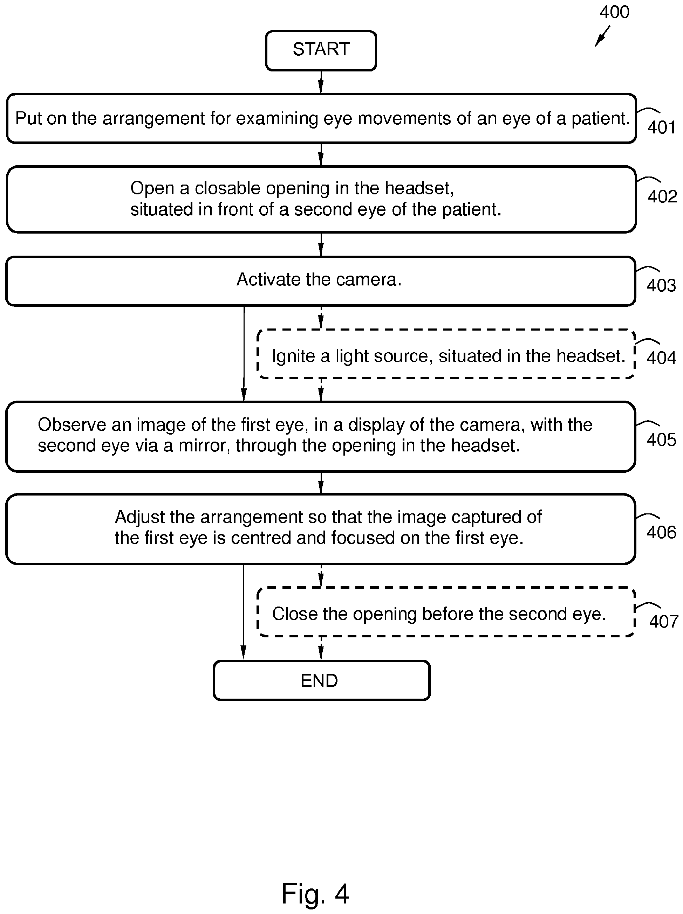

**Figure 4** illustrates an example of a method **400** for calibrating an arrangement 100 for enabling examination of eye movements of a first eye 111 of a patient via a camera 120.

In order to be able to calibrate the arrangement 100 correctly, the method 400 may comprise a number of steps **401-407.** However, some of these steps 401-407 may be performed solely in some alternative embodiments, like e.g. step 401-407. Further, the described steps 401-407 may be performed in a somewhat different chronological order than the numbering suggests. The method 400 may comprise the subsequent steps:
**Step 401** comprises positioning the arrangement 100 on the head 110 of the patient. Before the headset 130 is placed on the head 110, the patient may place the camera 120, or mobile telephone, in position for capturing images of the eye 111.
**Step 402** comprises opening a closable opening 135 in the headset 130, situated in front of a second eye of the patient; or, as may be the case, ascertain that the closable opening 135 is open.
**Step 403** comprises activating the camera 120. The camera 120 may be a camera sensor, a video camera, an infrared camera, etc., which may be comprised in a mobile phone, such as e.g. the mobile phone of the patient.
**Step 404,** which only may be performed in some alternative embodiments, comprises igniting a light source 150, situated in the headset 130.
   The light source 150 may be situated in the headset 130. The light source 150 may be configured to illuminate the first eye 111 of the patient.
**Step 405** comprises observing an image of the first eye 111, in a display 125 of the camera 120, with the second eye via a mirror 200, through the opening 135 in the headset 130.
**Step 406** comprises adjusting the arrangement 100 so that the image captured by the camera 120 of the first eye 111 is centred and focused on the first eye 111.
**Step 407,** which may be performed only in some embodiments, comprises closing the opening 135 in the headset 130 before the second eye to make sure the patient is not able to focus any of his/ her eyes.

Thereafter, the investigation can start. The investigation may be performed by playing vocal instructions of different movements, e.g. replayed by the cellular telephone of the patient via an app.

The arrangement 100 may be adjusted by fixating the camera 120 in a position where a lens of the camera 120 is directed towards the first eye 111 of the patient, at a distance d from the first eye 111. The fixation may be made by adjusting a mounting device 137, keeping the camera 120 in position.

Further, the adjustment may comprise fitting the headset 130 on the head 110 of the patient. Such adjustment may comprise adjusting straps holding the headset 130 in position on the head 110.

The terminology used in the description of the embodiments as illustrated in the accompanying drawings is not intended to be limiting of the described method 400 and arrangement 100. Various changes, substitutions or alterations may be made, without departing from invention embodiments as defined by the appended claims.

As used herein, the term "and/ or" comprises any and all combinations of one or more of the associated listed items. The term "or" as used herein, is to be interpreted as a mathematical OR, i.e., as an inclusive disjunction; not as a mathematical exclusive OR (XOR), unless expressly stated otherwise. In addition, the singular forms "a", "an" and "the" are to be interpreted as "at least one", thus also possibly comprising a plurality of entities of the same kind, unless expressly stated otherwise. It will be further understood that the terms "includes", "comprises", "including" or "comprising", specifies the presence of stated features, actions, integers, steps, operations, elements, or components, but do not preclude the presence or addition of one or more other features, actions, integers, steps, operations, elements, components, or groups thereof. A single unit such as e.g. a processor may fulfil the functions of several items recited in the claims. The mere fact that certain measures are recited in mutually different dependent claims does not indicate that a combination of these measures cannot be used to advantage. A computer program may be stored/ distributed on a suitable medium, such as an optical storage medium or a solid-state medium supplied together with or as part of other hardware, but may also be distributed in other forms such as via Internet or other wired or wireless communication system.

## Claims

1. An arrangement (100) for examining eye movements of a first eye (111) of a patient via a camera (120), by capturing a sequence of pictures of the first eye (111) and provide them to a distant physician via a mobile telephone, which arrangement (100) comprises:
a mobile telephone for providing pictures to a distant physician, the mobile telephone comprising a display and J Z a camera (120), configured to take a sequence of pictures of the first eye (111);
a headset (130), configured to fit on a head (110) of the patient and to fixate the camera (120) in a position where a lens of the camera (120) is directed towards the first eye (111) of the patient, at a distance (d) from the first eye (111);
wherein the headset (130) further comprises a closable opening (135) in front of a second eye of the patient, which second eye is not examined by the camera (120); and
the headset (130) comprises a mounting device (137), configured to fixate the mobile telephone (120) so that the display (125) is visible for the patient when looking in a mirror (200) with the second eye through the closable opening (135) in the headset (130).

2. The arrangement (100) according to claim 1, wherein the arrangement (100) further comprises:
a light source (150), situated in the headset (130), configured to illuminate the first eye (111) of the patient; and
an energy source (160), for feeding the light source (150) with current.

3. The arrangement (100) according to any of claim 1 or claim 2, wherein the headset (130) comprises a truncated cone (140), to be situated in front of the first eye (111) of the patient with the wide opening of the truncated cone (140) directed towards the first eye (111) and the narrow opening of the truncated cone (140) directed towards the lens of the camera (120).

4. The arrangement (100) according to any of claims 1-3, wherein the headset (130) is configured to be attached to the patient's head (110), screening off external light from falling into the first eye (111) of the patient.

5. A method (400) for calibrating an arrangement (100) according to any of claims 1-4, for enabling examination of eye movements of a first eye (111) of a patient via a camera (120), by capturing a sequence of images of the first eye (111) and provide them to a distant physician via a mobile telephone, which method (400) comprises the steps of:
positioning (401) the arrangement (100) according to any of claims 1-4 on the head (110) of the patient;
opening (402) a closable opening (135) in the headset (130), situated in front of a second eye of the patient;
activating (403) the camera (120);
observing (405) an image of the first eye (111), in a display (125) of the camera (120), with the second eye via a mirror (200), through the opening (135) in the headset (130); and
adjusting (406) the arrangement (100) so that the image captured of the first eye (111) is centred and focused on the first eye (111).

6. The method (400) according to claim 5, further comprising:
igniting (404) a light source (150), situated in the headset (130).

7. The method (400) according to any of claim 5 or claim 6, wherein the arrangement (100) is adjusted (406) by fixating the camera (120) in a position where a lens of the camera (120) is directed towards the first eye (111) of the patient, at a distance (d) from the first eye (111).

8. The method (400) according to any of claims 5-7, wherein the arrangement (100) is adjusted (406) by fitting the headset (130) on a head (110) of the patient.

## Patentansprüche

1. Eine Vorrichtung (100) zum Untersuchen von Augenbewegungen eines ersten Auges (111) eines Patienten über eine Kamera (120), indem eine Sequenz von Bildern des ersten Auges (111) aufgenommen wird und diese einem Arzt, der über ein Mobiltelefon zur Fernbehandlung zur Verfügung gestellt werden, wobei die Vorrichtung (100) umfasst:
ein Mobiltelefon zum Bereitstellen von Bildern an einen Arzt, der entfernt ist, wobei das Mobiltelefon ein Display und eine Kamera (120) umfasst, die konfiguriert ist, um eine Sequenz von Bildern des ersten Auges (111) aufzunehmen;
ein Headset (130), das so konfiguriert ist, dass es auf einen Kopf (110) des Patienten passt und die Kamera (120) in einer Position fixiert, in der eine Linse der Kamera (120) auf das erste Auge (111) des Patienten gerichtet ist, in einem Abstand (d) von dem ersten Auge (111);
wobei das Headset (130) weiterhin eine verschließbare Öffnung (135) vor einem zweiten Auge des Patienten aufweist, welches zweite Auge nicht von der Kamera (120) untersucht wird; und
das Headset (130) eine Befestigungsvorrichtung (137) umfasst, die so konfiguriert ist, dass sie das Mobiltelefon (120) so fixiert, dass die Anzeige (125) für den Patienten sichtbar ist, wenn er mit dem zweiten Auge durch die verschließbare Öffnung (135) im Headset (130) in einen Spiegel (200) schaut.

2. Die Vorrichtung (100) nach Anspruch 1, wobei die Vorrichtung (100) weiterhin Folgendes umfasst:
eine Lichtquelle (150), die sich in dem Headset (130) befindet und so konfiguriert ist, dass sie das erste Auge (111) des Patienten beleuchtet; und
eine Energiequelle (160), um die Lichtquelle (150) mit Strom zu versorgen.

3. Die Vorrichtung (100) nach Anspruch 1 oder 2, wobei das Headset (130) einen Kegelstumpf (140) umfasst, der vor dem ersten Auge (111) des Patienten anzuordnen ist, wobei die breite Öffnung des Kegelstumpfs (140) auf das erste Auge (111) und die schmale Öffnung des Kegelstumpfs (140) auf die Linse der Kamera (120) gerichtet ist.

4. Die Vorrichtung (100) nach einem der Ansprüche 1 bis 3, wobei das Headset (130) so konfiguriert ist, dass es am Kopf (110) des Patienten angebracht wird und externes Licht davon abschirmt, in das erste Auge (111) des Patienten zu gelangen.

5. Ein Verfahren (400) zum Kalibrieren einer Vorrichtung (100) gemäß einem der Ansprüche 1 bis 4, um eine Untersuchung von Augenbewegungen eines ersten Auges (111) eines Patienten über eine Kamera (120) zu ermöglichen, indem eine Sequenz von Bildern des ersten Auges (111) erfasst wird und diese einem entfernten Arzt über ein Mobiltelefon zur Verfügung gestellt werden, wobei das Verfahren (400) die folgenden Schritte umfasst:
Positionieren (401) der Vorrichtung (100) nach einem der Ansprüche 1 bis 4 auf dem Kopf (110) des Patienten;
Öffnen (402) einer verschließbaren Öffnung (135) in dem Headset (130), die sich vor einem zweiten Auge des Patienten befindet;
Aktivieren (403) der Kamera (120);
Betrachten (405) eines Bildes des ersten Auges (111) in einer Anzeige (125) der Kamera (120) mit dem zweiten Auge über einen Spiegel (200) durch die Öffnung (135) in dem Kopfhörer (130);
und Einstellen (406) der Vorrichtung (100), so dass das aufgenommene Bild des ersten Auges (111) zentriert und auf das erste Auge (111) fokussiert ist.

6. Verfahren (400) nach Anspruch 5, weiter umfassend: Zünden (404) einer Lichtquelle (150), die sich in dem Kopfhörer (130) befindet.

7. Verfahren (400) nach Anspruch 5 oder 6, wobei die Vorrichtung (100) durch Fixieren der Kamera (120) in einer Position eingestellt (406) wird, in der eine Linse der Kamera (120) auf das erste Auge (111) des Patienten gerichtet ist, und zwar in einem Abstand (d) von dem ersten Auge (111).

8. Verfahren (400) nach einem der Ansprüche 5 bis 7, wobei die Vorrichtung (100) durch Aufsetzen des Headsets (130) auf einen Kopf (110) des Patienten eingestellt wird (406).

## Revendications

1. Dispositif (100) servant à examiner les mouvements oculaires d'un premier oeil (111) d'un patient par l'intermédiaire d'une caméra (120), en capturant une séquence d'images du premier oeil (111) et en les fournissant à un médecin distant par l'intermédiaire d'un téléphone mobile, lequel dispositif (100) comprend :
un téléphone mobile pour fournir des images à un médecin distant, le téléphone mobile comprenant un écran et une caméra (120), configurés pour prendre une séquence d'images du premier oeil (111) ;
un casque (130), configuré pour se poser sur la tête (110) du patient et pour fixer la caméra (120) dans une position où la lentille de cette caméra (120) est dirigée vers le premier oeil (111) du patient, à une distance (d) du premier oeil (111) ;
sachant que le casque (130) comprend en outre une ouverture pouvant être fermée (135) face au deuxième oeil du patient, lequel deuxième oeil n'est pas examiné par la caméra (120) ; et que
le casque (130) comprend un dispositif de montage (137), configuré pour fixer le téléphone mobile (120) de sorte que l'écran (125) soit visible pour le patient lorsqu'il regarde dans un miroir (200) avec le deuxième oeil à travers l'ouverture pouvant être fermée (135) dans le casque (130).

2. Dispositif (100) selon la revendication 1, dans lequel le dispositif (100) comprend en outre :
une source de lumière (150), située dans le casque (130), configurée pour éclairer le premier oeil (111) du patient ; et
une source d'énergie (160), pour alimenter la source de lumière (150) en courant.

3. Dispositif (100) selon l'une quelconque des revendications 1 ou 2, dans lequel le casque (130) comprend un cône tronqué (140), destiné à être situé devant le premier oeil (111) du patient, l'ouverture large du cône tronqué (140) étant dirigée vers le premier oeil (111) et l'ouverture étroite du cône tronqué (140) étant dirigée vers l'objectif de la caméra (120).

4. Dispositif (100) selon l'une quelconque des revendications 1 à 3, dans lequel le casque (130) est configuré pour être fixé sur la tête du patient (110), empêchant la lumière extérieure de tomber dans le premier oeil (111) du patient.

5. Procédé (400) pour calibrer un dispositif (100) selon l'une quelconque des revendications 1 à 4, pour permettre l'examen des mouvements oculaires d'un premier oeil (111) d'un patient via une caméra (120), en capturant une séquence d'images du premier oeil (111) et en les fournissant à un médecin distant via un téléphone mobile, lequel procédé (400) comprend les étapes suivantes :
positionnement (401) du dispositif (100) selon l'une quelconque des revendications 1 à 4 sur la tête (110) du patient ;
ouverture (402) d'un orifice pouvant se fermer (135) dans le casque (130), situé en face du deuxième oeil du patient ;
activation (403) de la caméra (120) ;
observation (405) d'une image du premier oeil (111), dans un affichage (125) de la caméra (120), le second oeil étant via un miroir (200), à travers l'ouverture (135) dans le casque (130) ; et
réglage (406) de l'dispositif (100) de sorte que l'image capturée du premier oeil (111) soit centrée et focalisée sur le premier oeil (111).

6. Procédé (400) selon la revendication 5, comprenant en outre :
l'allumage (404) une source de lumière (150), située dans le casque (130).

7. Procédé (400) selon l'une quelconque des revendications 5 ou 6, dans lequel le dispositif (100) est réglé (406) en fixant la caméra (120) dans une position où un objectif de la caméra (120) est dirigé vers le premier oeil (111) du patient, à une distance (d) du premier oeil (111).

8. Procédé (400) selon l'une quelconque des revendications 5 à 7, dans lequel le dispositif (100) est réglé (406) en adaptant le casque (130) sur la tête (110) du patient.
